# EUROPEAN PATENT APPLICATION

(11) **EP 2 277 482 A1**
(43) Date of publication of application: **26.01.2011**
(21) Application number: 09746454.9
(22) Date of filing: 07.04.2009
(51) Int. Cl.: A61F 13/15, A61F 13/472, A61F 13/534, A61F 13/539

(54) **ABSORPTIVE ARTICLE**

(30) Priority: 15.05.2008 JP 2008128796
(71) Applicant: Unicharm Corporation, Ehime 799-0111 (JP)
(72) Inventor: SAGISAKA, Minako, Kanonji-shi Kagawa 769-1602 (JP); NISHITANI, Kazuya, Kanonji-shi Kagawa 769-1602 (JP)
(74) Representative: Knights, Rupert
(86) International application number: PCT/JP2009/057416
(87) International publication number: WO 2009/139247

(57) **Abstract**

To provide an absorbent article free from generation of twisting and capable of always realizing a stable deformed state.

An absorbent article comprising at least a liquid-permeable sheet, a liquid-impermeable sheet, and an absorber sandwiched between the liquid-permeable sheet and the liquid-impermeable sheet, wherein:
the absorber consists of an absorbing layer center part and an absorbing part formed in the periphery of the absorbing layer center part to have a lower basis weight than the absorbing layer center part;
on the skin-contact surface side of the absorbent article, compressed grooves are provided along at least widthwise right and left side edges to surround the absorbing layer center part, the compressed groove having an intermittent portion in the vicinity of the longitudinal centerline; and
on the skin-non-contact surface side of the absorber, a space part being nearly convex toward the skin-contact surface side and formed between the absorber and the liquid-impermeable sheet, is present in the side part of at least a part of compressed groove out of the compressed grooves provided along widthwise right and left side edges.

## Description

### TECHNICAL FIELD

The present invention relates to an absorbent article. More specifically, the present invention relates to an absorbent article such as sanitary napkin and incontinence pad, in which a compressed groove is provided at a predetermined position of an absorber and a space part is provided in the side part of the compressed groove.

### BACKGROUND ART

As for the absorbent article aiming at preventing leakage of a body fluid, an absorbent article in which a three-dimensionally shaped protrusion is formed on the skin-contact surface and the periphery of the protrusion is continuously surrounded by a concave part, is known (see, Japanese Unexamined Patent Publication (Kokai) No. 11-33054).

However, in such an absorbent article where the periphery of the protrusion is continuously surrounded by a concaved groove and the groove is formed by compressing a surface sheet and an adsorbing layer, the groove part has a higher density than the periphery and in turn the rigidity is increased, which makes it difficult to flexibly deform following the body shape. In particular, in the rear end of the protrusion, which corresponds to the backward of the excretory part, the groove continues in the width direction of the absorber. Therefore, when a force is applied from the widthwise outside on fitting, the absorbent article can be hardly raised toward the wearer's groin. Even when raised, since the structure is not designed to specify the position of the wearer's groin, the raised portion may not be at a position along the shape of the wearer' groin and this induces generation of twisting. Furthermore, since the groove part is disposed between the nearly center part in the thickness direction of the core part and the back surface of the core part, when a compressive force is applied in the width direction from the wearer's femoral region, the force transmitted to the core part through the groove diffuses from the skin-contact surface side in the thickness direction of the absorber to various directions such as skin-non-contact surface side. As a result, the core part may be bent in various directions, and it is impossible to always obtain a stable deformed state.

Also, for improving the leakage in the width direction and the instability of surface shape of the absorbing layer on fitting, an absorbent article where a groove part is separated into a plurality of parts has been reported (see, Japanese Unexamined Patent Publication (Kokai) No. 2007-195665). However, in this case, since the groove part is provided only in the absorbing layer on the skin-contact surface side, the absorber may be bent in various directions, and it is impossible to always obtain a stable deformed state.

On the other hand, as for an absorbent article having a flexible axis not only in the absorbing layer on the skin-contact surface side but also in the absorbing layer on the skin-non-contact surface side, an absorbent article where a three-dimensional groove as a concave part is provided on the skin-non-contact surface side by applying a grooving work from the skin-non-contact surface side toward the skin-contact surface side, has been reported (see, Japanese Unexamined Patent Publication (Kokai) No. 10-99372). The three-dimensional groove is formed only on either one side, inside or outside, of the position on the skin-non-contact surface side corresponding to the three-dimensional groove on the skin-contact surface side. However, the absorbent article takes various fitting shapes when fitted to groin. Therefore, depending on the fitting form, it is difficult for the absorbent article of Japanese Unexamined Patent Publication (Kokai) No. 10-99372 to always take a predetermined shape ensuring excellent fitting property.

### SUMMARY OF THE INVENTION

Accordingly, an object of the present invention is to provide an absorbent article free from generation of twisting and capable of eliminating a fear of bending of the absorbing layer center part (core part) in various directions and always realizing a stable deformed state.

Under these circumstances, the present inventors have made intensive studies, as a result, it has been found that when on the skin-contact surface side, a compressed groove is provided to surround the absorbing layer center part, the compressed groove being separated at a specific position, and on the skin-non-contact surface side, a nearly convex space part is formed in the side part of at least a part of a compressed groove and located between the absorber and the liquid-impermeable sheet, a protruding part formed on or inside of the skin-non-contact surface in the thickness direction of the absorbent article readily enters the space part; and the above-described object can be thereby attained. The present invention has been accomplished based on this finding.

That is,
(1) the present invention provides an absorbent article comprising at least a liquid-permeable sheet, a liquid-impermeable sheet, and an absorber sandwiched between the liquid-permeable sheet and the liquid-impermeable sheet, wherein:
   the absorber consists of an absorbing layer center part and an absorbing part formed in the periphery of the absorbing layer center part to have a lower basis weight compared to the absorbing layer center part;
   on the skin-contact surface side of the absorbent article, compressed grooves are provided along at least widthwise right and left side edges to surround the absorbing layer center part, the compressed groove having an intermittent portion in the vicinity of the longitudinal centerline; and
   on the skin-non-contact surface side of the absorber, a space part being nearly convex toward the skin-contact surface side and formed between the absorber and the liquid-impermeable sheet, is present in the side part of at least a part of a compressed groove out of the compressed grooves provided along widthwise right and left side edges.
(2) The present invention provides the absorbent article as described in (1), wherein the intermittent portion is formed on the longitudinal back side.
(3) The present invention provides the absorbent article as described in (2), wherein the intermittent portion is further formed on the longitudinal front side.
(4) The present invention provides the absorbent article as described in any one of (1) to (3), wherein the space part is formed on both side parts of at least a part of a compressed groove out of the compressed grooves provided along widthwise right and left side edges.
(5) The present invention provides the absorbent article as described in any one of (1) to (4), wherein the space part is formed to extend to both ends of each compressed groove provided along widthwise right and left side edges.

According to the absorbent article of the present invention, an intermittent portion is provided near the longitudinal centerline, so that the absorbing layer center part can be raised toward the wearer's body using the intermittent portion as the base point, and generation of twisting can be thereby prevented.

Also, on the skin-non-contact surface side of the absorber, a space part is provided in the side part of the compressed groove, so that the protruding part provided on or inside of the skin-non-contact surface of the absorber can move to be pushed into the space part depending on the fitted state of the absorbent article, and push up the absorbing layer center part from the skin-non-contact surface side, and in turn the absorbent article can be always stably contacted to the wearer's excretory part to prevent leakage of the excreta.

### BRIEF DESCRIPTION OF THE DRAWINGS

Fig. 1 is a plan view of a representative absorbent article of the present invention.
Fig. 2 is an A-A' cross-sectional view of Fig. 1.
Fig. 3 is an enlarged view of the circled region of Fig. 2.
Fig. 4 is a plan view of an embodiment of the absorbent article of the present invention.
Fig. 5 is a plan view of another embodiment of the absorbent article of the present invention.
Fig. 6 is a plan view of another embodiment of the absorbent article of the present invention.
Fig. 7 is a plan view of another embodiment of the absorbent article of the present invention.
Fig. 8 is a plan view illustrating one embodiment of the space part 10 region of the absorbent article of Fig. 1 from the skin-non-contact surface side.

### EMBODIMENT FOR CARRYING OUT THE INVENTION

The present invention is described below by referring to the drawings, but the present invention is not limited to those illustrated in the drawings.

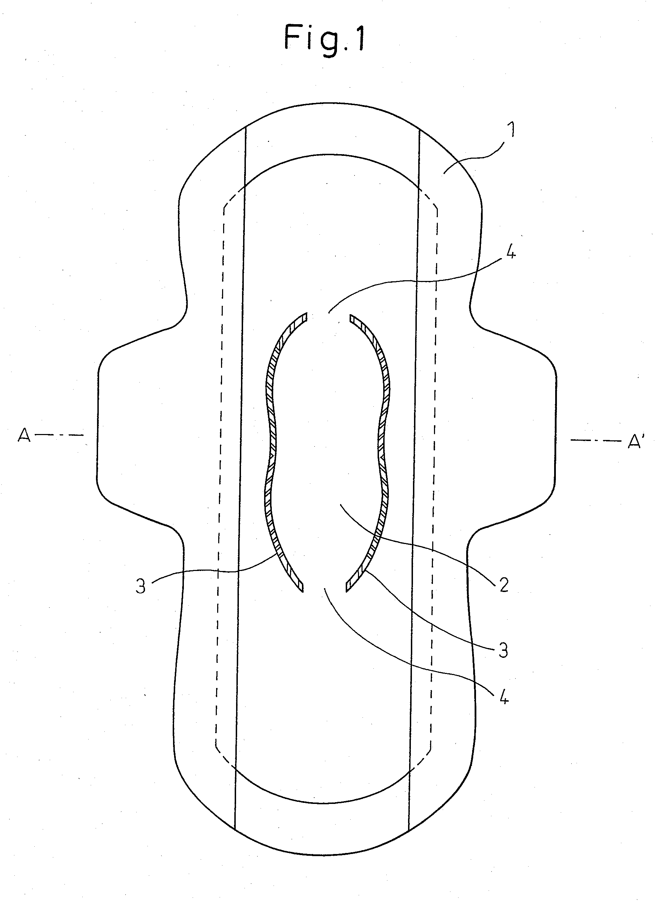

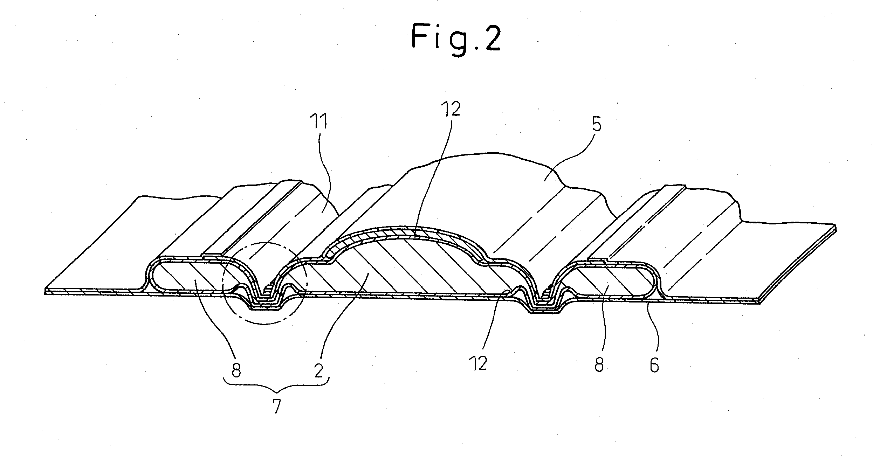

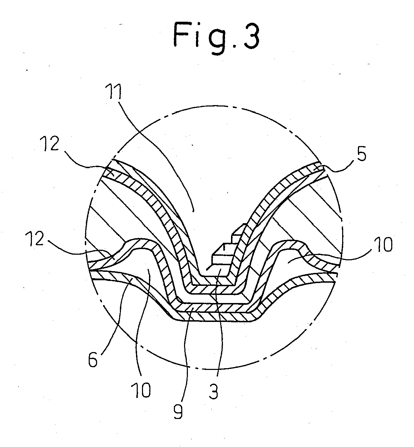

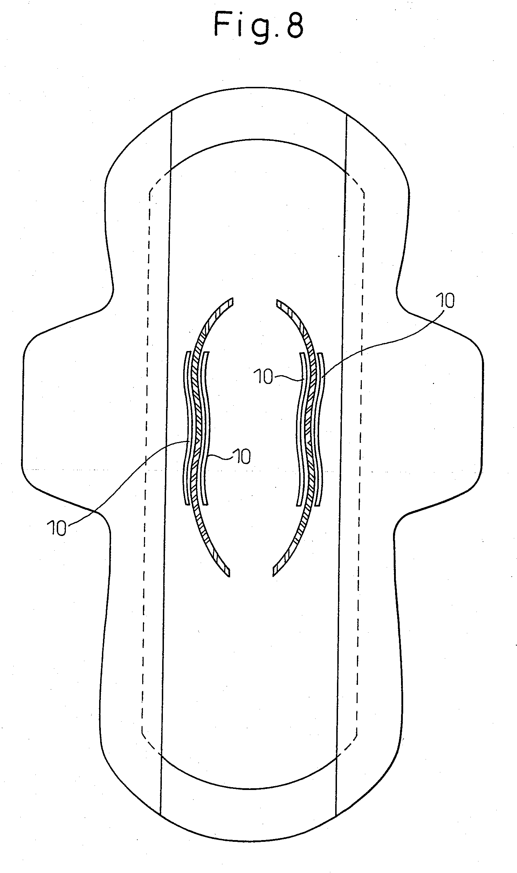

Fig. 1 is a plan view of a representative absorbent article 1 of the present invention; Fig. 2 is an A-A' cross-sectional view of Fig. 1; Fig. 3 is an enlarged view of the circled region in Fig. 2; Figs. 4 to 7 are plan views illustrating the embodiments of the absorbent article 1 of the present invention; and Fig. 8 is a plan view illustrating one embodiment of the space part 10 region of the absorbent article of Fig. 1 from the skin-non-contact surface side.

Incidentally, in the absorbent articles 1 of the present invention illustrated in these drawings, a pair of wing parts extending from both side edges in the longitudinal direction are provided, but the wing part may not be provided.

The absorbent article 1 of the present invention consists of at least a liquid-permeable sheet 5, a liquid-impermeable sheet 6, and an absorber 7. The absorber 7 is usually integrated with a mount 12, and the integrated absorber is sandwiched between the liquid-permeable sheet 5 and the liquid-impermeable sheet 6. The absorber 7 is composed of an absorbing layer center part 2 and an absorbing part 8 in the periphery thereof, and the peripheral absorbing part 8 is formed to have a lower basis weight compared to the absorbing layer center part 2. Although it may vary depending on the usage or the like of the absorber, the basis weight of the absorbing layer center part 2 is generally from 200 to 1,200 g/m², and the basis weight of the peripheral absorbing part 8 is from 100 to 400 g/m². The absorber 7 is not necessarily required to be composed of one absorbing layer, but may be divided into, for example, an absorbing part as the lower layer and a protrusion as the upper layer.

As illustrated in Fig. 1, in the absorbent article 1 of the present invention, when planarly viewed, compressed grooves 3 are provided along at least side edges on the right and left sides in the width direction to surround the absorbing layer center part 2 (hereinafter, these compressed grooves are referred to as a "widthwise right side edge compressed groove" and a "widthwise left side edge compressed groove", respectively, and these are sometimes collectively referred to as "widthwise right and left side edge compressed grooves"). The liquid-permeable sheet 5 and the absorber 7 are joined together by the compressed grooves 3.

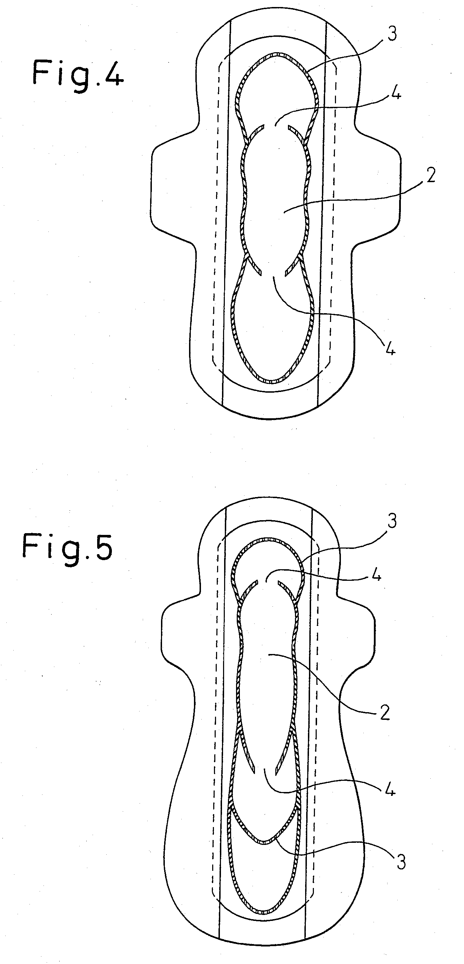

The shape of the compressed groove 3 may be sufficient if it is a shape surrounding the absorbing layer center part 2, and examples thereof include a waved shape, a linear shape, a widthwise outwardly convex shape and a widthwise inwardly convex shape. Also, the shape of the compressed groove 3 may be, as illustrated in Figs. 4 to 7, a shape surrounding the absorbing layer center part 2 and further being longitudinally outwardly convex on the longitudinal front and back sides. However, the widthwise right and left side edge compressed grooves 3 each is preferably in a shape of its front and rear ends extending toward the absorbing layer center part 2. Fig. 1 illustrates an embodiment where front and rear ends of each of the widthwise right and left side edge compressed grooves 3 are slightly curved toward the absorbing layer center part 2. As used herein, the term "end" of the compressed groove means a terminal portion of each of the widthwise right and left side edge compressed grooves 3. When the widthwise right and left side edge compressed grooves each takes a shape of its front and rear ends extending toward the absorbing layer center part, even if excreta diffuses in the width direction in the absorbing layer center part and reaches the compressed groove, the flow of excreta can be led to the absorbing layer center part 2 along the compressed groove and the absorbing surface can be effectively used, so that the leakage of excreta can be suppressed.

The shape of the groove, per se, in the compressed groove 3 is not particularly limited, and may be, for example, a concave part where the entire compressed groove has a constant depth, but as illustrated in Fig. 3, the groove is preferably formed as an aggregate composed of a deepest part and a shallow part. When such a shape is taken, by virtue of the difference between the density in the deepest part and the density in the shallow part, effective in draft of excreta can be attained and not only the rigidity is prevented from becoming excessively high but also the texture is hardly impaired.

The shape of the absorbing layer center part 2 surrounded by compressed groves 3 includes, but is not limited to rectangular, elliptical, circular, droplet-like, quadrangular and triangular shapes. The dimension of the absorbing layer center part 2 surrounded by the compressed grooves 3 is preferably about 40 mm or more, more preferably from about 60 to about 120 mm, in the longitudinal direction, and preferably about 10 mm or more, more preferably from about 25 to about 50 mm, in the width direction. As for the positional relationship of the absorbing layer center part 2 surrounded by the compressed grooves 3, usually, the longitudinal front end of the absorbing layer center part 2 surrounded by the compressed grooves 3 is present at least 10 mm backward from the longitudinal front end of the absorber 7, and the longitudinal rear end of the absorbing layer center part 2 surrounded by the compressed grooves 3 is present at least 10 mm frontward from the longitudinal rear end of the absorber 7.

The compressed groove 3 has an intermittent portion 4 where the absorber is not compressed, in the vicinity of the longitudinal centerline. When a force is applied from the widthwise outside of the absorber on fitting, the intermittent portion is flexibly bent due to difference in rigidity between the compressed groove and the intermittent portion, as a result, the absorbing layer center part and compressed grooves provided in the width direction are raised to the wearer's body. Furthermore, an intermittent portion is provided in the vicinity of the longitudinal centerline, and therefore the absorbing layer center part and compressed grooves provided in the width direction are inevitably raised toward the wearer's body (cleft of buttocks) and are not raised at a position other than on the longitudinal centerline. The absorbent article can be thereby prevented from occurrence of twisting.

The intermittent portion 4 may be provided on either one side or both sides of the front side and the back side in the longitudinal direction, but is preferably provided at least on the longitudinal back side. Because the absorbing layer center part is more easily fit to the body, although a gap is readily produced between the wearer's body and the absorbent article when fitting the absorbent article. In Fig. 1, an embodiment where an intermittent portion is provided on front and back both sides in the longitudinal direction is illustrated.

The length of the intermittent portion is usually from about 2 to about 40 mm, preferably from about 5 to about 15 mm. If the length of the intermittent portion is less than 2 mm, a low-rigidity portion cannot be formed and the absorbent article can hardly follow the movement of the body, and therefore this is disadvantage while, if it exceeds 40 mm, not only the leakage-preventing property is decreased but also heavy twisting is generated due to wide movement of the absorbing layer center part, and therefore this is disadvantageous.

The compressed groove 3 forms a protruding part 9 on the skin-non-contact surface side. The protruding part 9 is usually formed such that the protruding part 9 is at the same position as the skin-contact surface in the thickness direction or a position inside of the skin-contact surface in a state of the absorbent article being not fitted and protrudes from the skin-non-contact surface to the skin-non-conduct surface side in a fitted state, but the protruding part 9 is preferably formed to protrude from the skin-non-contact surface to the skin-non-contact surface side already in a non-fitted state.

In the side part of at least a part of each of the widthwise right and left side edge compressed grooves 3, a space part 10 nearly convex toward the skin-contact surface side is formed between the mount 12 and the liquid-impermeable sheet 6 such that the space part almost sandwiches the protruding part 9 when cross-sectionally viewed. As used herein, the term "side part" means a region in the vicinity of each compressed groove, including the side edge part of each of the widthwise right and left side edge compressed grooves 3. When a compressive force is applied from the wearer's femoral region, the protruding part 9 deforms and moves to the widthwise inner side, and the space part 10 is a portion into which the deformed and moved protruding part 9 is pushed. The thus-moved and pushed-in protruding part 9 pushes up the absorbing layer center part 2 from the skin-non-contact surface side of the absorbing layer center part 2, whereby the absorbing layer center part 2 deforms to protrude to the skin-contact surface side and closely contacts with the wearer's excretory part ("engagement effect").

In Fig. 2 and in Fig. 3 enlarging the circled region of Fig. 2, an embodiment where the protruding part 9 protrudes from the skin-non-contact surface to the skin-non-contact surface side in a non-fitted state is illustrated.

In the case where the protruding part 9 protrudes from the skin-non-contact surface to the skin-non-contact surface side in a fitted state or where the protruding part 9 is formed to protrude from the skin-non-contact surface to the skin-non-contact surface side already in a non-fitted state, the protrusion degree of the protruding part 9 is to such an extent as allowing the bottom of the compressed groove 3 to protrude to the skin-non-contact surface side in a range of about 0.5 to about 10 mm from the skin-non-contact surface of the absorbing layer center part 2. That is, the protruding part protrudes such that the difference of height in the thickness direction between the bottom of the compressed groove 3 and the skin-non-contact surface of the absorbing layer center part 2 becomes from about 0.5 to about 10 mm. This difference is preferably about 1 mm. When this difference is from about 0.5 to about 10 mm, the protruding part 9 can easily enter the space part 10.

The space part 10 is formed to locate its top closer to the skin-contact surface side by about 0.5 to about 10 mm than the skin-non-contact surface of the absorbing layer center part 2. That is, the space part is formed such that the difference of height in the thickness direction of the absorber 7 between the bottom of the compressed groove 3 and the top of the space part 10 becomes from about 0.5 to about 10 mm. This difference is preferably about 2 mm. When this difference is from about 0.5 to about 10 mm, the protruding part 9 can easily enter the space part 10.

For example, as illustrated in Fig. 8, the space part 10 is sufficient if it is provided in at least a part of each of the widthwise right and left side edge compressed grooves 3, and the region thereof is not particularly limited. However, the space part 10 needs to be formed at least in a portion subject to a lateral pressure from legs. Therefore in the case where the absorbent article is a sanitary napkin, the space part 10 is preferably formed at least in a region that comes into contact with ostium vaginae. In this case, the length in the longitudinal direction of the space part 10 is at least 15 mm or more. If the length is less than 15 mm, the "engagement effect" can be hardly obtained. In the absorbent article of the present invention, the space part 10 is preferably formed to extend to both ends of each of the widthwise right and left side edge compressed grooves 3 (not shown), because the flow of excreta can be led to the absorbing layer center part.

The space part 10 is provided in at least a part of each of the widthwise right and left side edge compressed grooves 3, but need not be always provided in both side parts of the compressed groove.

For example, the space part 10 may be formed in only one side part of each compressed groove 3, that is, may be formed in one side part of the widthwise left side edge compressed groove 3 and one side part of the widthwise right side edge compressed groove 3 to make a pair and be located between the widthwise left side edge compressed groove 3 and the widthwise right side edge compressed groove 3. By such a construction, when a force is laterally applied on fitting, the absorbing layer center part 2 is lifted to the wearer side using the space part 10 as the base point and on the other hand, the protruding part 9 deforms and moves to the widthwise inner side to push up and raise the absorbing center part 2, allowing the absorbing layer center part 2 to readily fit to the body. In this case, the pair of space parts 10 are preferably disposed at a position closer to respective compressed grooves 3 than to the longitudinal centerline, whereby a stable deformed form can be ensured.

Preferably, the space part 10 is provided in both side parts of each compressed groove 3. Because when a lateral pressure is applied, the protruding part moves nearly in parallel to the absorbing layer center part side to allow a force to be easily transmitted in a constant direction from the compressed groove to the absorbing layer center part and in turn the shape of the absorbing layer center part can be maintained as a bilaterally symmetric chevron. Furthermore, by virtue of providing the space part also in the side part on the non-absorbing layer center part side of the compressed groove, the space part can unfailingly push up the absorber portion outside of the compressed groove and therefore, and therefore the excreta can be stemmed by the compressed groove without fail. In this way, formation of a space part in both side parts enables ensuring a more stable deformed form compared to the formation of a space part in one side part and enhancing the close contact to the skin. In Fig. 3, an embodiment where a space part 10 is provided in both side parts of a protruding part 9 to sandwich the protruding part is illustrated.

The compressed groove 3 has a pair of wall parts 11 formed by the engagement of an embossing apparatus described later (see, Figs. 2 and 3). For example, when the liquid-permeable sheet 5 and the absorber 7 are compressed to the thickness direction of the absorbent article 1, a strong tensile stress is generated in nearly the center part in the thickness direction of the absorbent article 1 and the absorber 7 is stretched by the tensile stress, whereby the wall part 11 is formed. The density of the wall part 11 formed resulting from the absorber 7 being stretched is low in nearly the center part thereof. This low-density portion can suppress diffusion of the excreta running from the high-density portion when the absorbing layer center part 2 absorbs the excreta.

Figs. 4 to 7 illustrate embodiments (first, second, third and fourth embodiments, respectively) of the absorbent article 1 of the present invention. In the first to fourth embodiments, in addition to the compressed grooves 3 illustrated in Fig. 1, a compressed groove 3 in a longitudinally outwardly convex shape is provided on the longitudinal front and back sides in the skin-contact surface side of the absorbent article 1. Also in the first to fourth embodiments, all compressed grooves are provided to exhibit symmetry with respect to the longitudinal centerline as the base point, similarly to the embodiment illustrated in Fig. 1.

In the first embodiment, as illustrated in Fig. 4, a compressed groove is further connected to the widthwise left side edge compressed groove 3 at a position where the nearly linear portion of the compressed groove is bent toward the absorbing layer center part 2 (curved position). While this compressed groove connected draws a longitudinally outwardly convex shape, the compressed groove is connected to the curved position of the widthwise right side edge compressed groove 3. Such a convex compressed groove is formed on front and back both sides in the longitudinal direction. In this embodiment, the compressed groove is provided in a closed chain form in the circumferential periphery of the entire absorber.

In this embodiment, the absorbing layer center part is raised using the intermittent portions as the base points, and symmetric deformation with respect to the base point assigned to the longitudinal centerline in the absorbing layer center part is transmitted as it is to the longitudinal front and back sides, whereby generation of twisting can be prevented. The compressed groove where the liquid-permeable sheet and the absorbing layer center part are compressed, has a higher density than the peripheral absorbing part and has a force of drawing excreta. Therefore, even if excreta is leaked from the absorbing layer center part, the excreta is led toward the compressed groove on the longitudinal front or back side, whereby leakage of excreta to the outside of the absorbent article can be prevented by efficiently utilizing the entire surface of the absorber.

In the second embodiment, as illustrated in Fig. 5, the compressed groove is provided in nearly the same closed chain form as in the first embodiment. However, in the second embodiment, the widthwise right and left side edge compressed grooves are formed at a position slightly closer to the longitudinal front side as compared with the first embodiment. Also, on the longitudinal back side of the widthwise right and left side edge compressed grooves, a compressed groove in a longitudinally outwardly convex shape is further formed inside of the compressed groove formed in a longitudinally outwardly convex shape in the first embodiment.

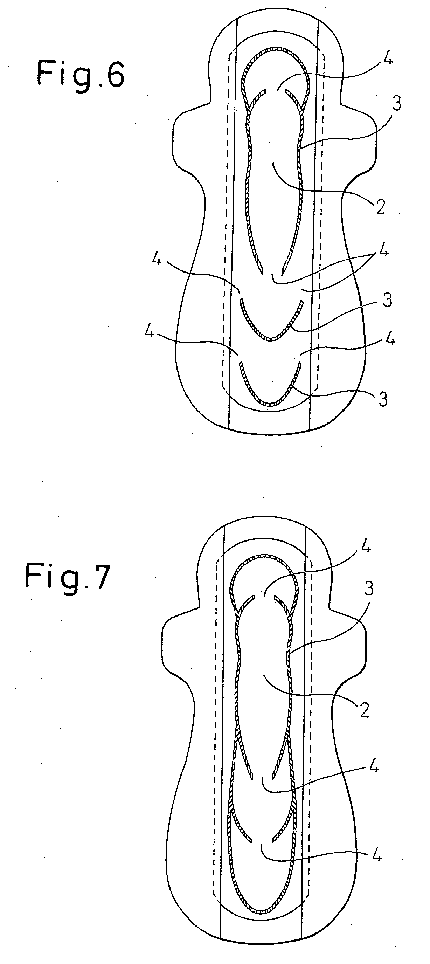

In the third embodiment, as illustrated in Fig. 6, the compressed groove in a closed chain form in the second embodiment is separated at the position having the longitudinal rear end of each of the widthwise right and left side edge compressed grooves 3. More specifically, on the longitudinal back side of the widthwise right and left side edge compressed grooves 3, two compressed grooves formed in a longitudinally outwardly convex shape each has an intermittent portion 4 in the widthwise left side edge and the widthwise right side edge.

In the fourth embodiment, as illustrated in Fig. 7, a compressed groove in a longitudinally outwardly convex shape formed between the widthwise right/left side edge compressed groove 3 and the compressed groove formed in a longitudinally outwardly convex shape in the first embodiment, has an intermittent portion 4 on the longitudinal centerline.

In the second to fourth embodiments, one or more compressed grooves are further provided on the longitudinal back side of the widthwise right and left side edge compressed grooves. Therefore, even if excreta is leaked from the absorbing layer center part, the excreta can be led to the compressed groove on the longitudinal back side. Furthermore, in the fourth embodiment, when a force is applied from the widthwise outside of the absorbent article, the absorber can be easily raised due to rigidity difference between the compressed groove and the intermittent portion.

In these first to fourth embodiments, the dimension of absorbing layer center part, the shape of groove, per se, of the compressed groove, the length of intermittent portion, the length of space part, the positional relationship between the top of space part and the bottom of compressed groove, and the difference of height in the thickness direction between the compressed groove and the skin-non-contact surface of absorbing layer center part, are the same as those in the embodiment of a representative absorbent article of the present invention illustrated in Fig. 1.

The liquid-permeable sheet is entirely or partially liquid-permeable, and the liquid permeation area is formed of, for example, a resin film having formed therein a large number of liquid permeation cells, a net-like sheet having a large number of meshes, a liquid-permeable nonwoven fabric or a woven fabric. As for the resin film or net-like sheet, those formed from polypropylene, polyethylene, polyethylene terephthalate or the like may be used. As to the nonwoven fabric, for example, a spunlaced nonwoven fiber formed from a cellulose fiber such as rayon, a synthetic resin fiber or the like, and an air-through nonwoven fiber formed from the synthetic resin fiber above may be used. Also, in combination with forming a large number of liquid permeation cells, a silicone-containing or fluorine-containing water-repellent oily agent may be coated on the liquid-permeable sheet to deter attachment of a body fluid to the outer surface.

The diameter of the fiber above is preferably from 1.1 to 6.6 dtex, and the basis weight of the liquid-permeable sheet is preferably from 15 to 120 g/m².

The absorber constituting the absorbent article of the present invention is composed of, for example, pulp, chemical pulp, rayon, acetate, natural cotton, polymer absorber, fibrous polymer absorber, synthetic fiber, foam or a mixture thereof, and its material that hardly loses shape and causes less chemical irritation is preferred. The material above is stacked, and the stack is covered by winding a sheet material (covering material) therearound or sandwiching the stack between top and bottom two sheets and then pressed to form an absorber. Examples of the covering material include a cellulose-based fiber such as pulp, chemical pulp, rayon, acetate and natural cotton, and a thermoplastic resin such as polyethylene, polypropylene and polyethylene terephthalate.

In the case where the absorber has a protrusion, the protrusion may be formed by disposing another absorber on an absorber composed of one or more layers or causing the basis weight to be partially higher than the periphery within a one-layer absorber.

The liquid-impermeable sheet is sparingly permeable to a liquid and does not pass a body fluid. Examples thereof include polyethylene, polypropylene, polyethylene terephthalate, polyvinyl alcohol, polylactic acid, polybutyl succinate, nonwoven fabric, paper and a laminated material thereof, each having a thickness of 15 to 60 µm. A breathable film obtained by filling an inorganic filler and applying stretching may also be used. Specific examples thereof include a film prepared using a low-density polyethylene resin as the main component and adjusted to have a basis weight of 15 to 30 g/m².

The absorbent article of the present invention can be used as a sanitary napkin, an incontinence pad or the like.

The production method of the absorbent article of the present invention comprises at least an embossing step of overlapping a liquid-permeable sheet 5 and an absorber 7 and of compressing these in the thickness direction to surround the absorbing layer center part 2. Thereby, a compressed groove 3 protruding from the skin-non-contact surface to the skin-non-contact surface side is formed. More specifically, in the embossing step, using an embossing apparatus comprising an upper roll part having on the surface thereof a convex part formed in a predetermined pattern and a lower roll part having formed on the surface thereof a concave part with which the convex part can engage, the absorbent material obtained by overlapping a liquid-permeable sheet 5 and an absorber 7 is disposed between the upper and lower roll parts arranged to engage with, such that the liquid-permeable sheet 5 is put into contact with the upper roll part, and the absorbent material is then compressed. If desired, a non-woven fabric or an air-laid pulp maybe inserted as a second sheet between the liquid-permeable sheet 5 and the absorber 7.

An adhesive layer, for example, a layer formed of a hot-melt resin or a hot-melt adhesive, may be provided between the liquid-permeable sheet 5 and the absorber 7 or between the second sheet and the absorber 7. In the case of providing a layer formed of a hot-melt resin or a hot-melt adhesive, the embossing work is preferably heat embossing. Simultaneously with forming a compressed groove by this embossing, the liquid-permeable sheet 5 and the absorber 7 may be integrated.

In the case of providing a wing part, for example, a wing member may be joined by inserting it between the liquid-permeable sheet 5 and the liquid-impermeable sheet 6, or the liquid-permeable sheet 5 may be extended to the width direction without disposing a wing member. Examples of the wing member include a nonwoven fabric and a film.

Finally, a liquid-impermeable sheet 6 is fed to the skin-non-contact surface side of the absorber 7, and the liquid-permeable sheet 5 and the liquid-impermeable sheet 6 are then joined together.

In the embodiment above, the production method of a sanitary napkin is described as an example, but the production method of the present invention can be applied to production of other absorbent articles such as incontinence pad.

## Claims

1. An absorbent article comprising at least a liquid-permeable sheet, a liquid-impermeable sheet, and an absorber sandwiched between said liquid-permeable sheet and said liquid-impermeable sheet, wherein:
said absorber consists of an absorbing layer center part and an absorbing part formed in the periphery of said absorbing layer center part to have a lower basis weight compared to said absorbing layer center part;
on the skin-contact surface side of said absorbent article, compressed grooves are provided along at least widthwise right and left side edges to surround said absorbing layer center part, said compressed groove having an intermittent portion in the vicinity of the longitudinal centerline; and
on the skin-non-contact surface side of said absorber, a space part being nearly convex toward the skin-contact surface side and formed between the absorber and the liquid-impermeable sheet, is present in the side part of at least a part of compressed groove out of the compressed grooves provided along widthwise right and left side edges.

2. The absorbent article according to claim 1, wherein said intermittent portion is formed on the longitudinal back side.

3. The absorbent article according to claim 2, wherein said intermittent portion is further formed on the longitudinal front side.

4. The absorbent article according to any one of claims 1 to 3, wherein said space part is formed on both side parts of at least a part of compressed groove out of the compressed grooves provided along widthwise right and left side edges.

5. The absorbent article according to any one of claims 1 to 4, wherein said space part is formed to extend to both ends of each compressed groove provided along widthwise right and left side edges.
